# DEMANDE DE BREVET EUROPEEN

(11) **EP 0 543 763 A1**
(43) Date de publication de la demande: **26.05.1993**
(21) Numéro de dépôt: 92480179.8
(22) Date de dépôt: 20.11.1992
(51) Int. Cl.: B65F 1/14, B65F 1/10, A61B 19/02

(54) **Réceptacle à accés personnalisé pour la collecte d'objets d'origine spécifique**

(30) Priorité: 21.11.1991 FR 9114613
(71) Demandeur: NICOLETTI AGREGATS S.A., F-06100 Nice (FR); SOGEDET SaRL, F-78000 Versailles (FR)
(72) Inventeur: Nicoletti, Didier, F-06200 Nice (FR); Donche, Jean-Pierre, F-06000 Nice (FR)
(74) Mandataire: Hammond, William

(57) **Abrégé**

Réceptacle à accès personnalisé pour la collecte contrôlée d'objets d'origine et de destination spécifiques.

Il comprend :
- un dispositif d'accès desdits objets sur autorisation après contrôle de l'identité du déposant et de la conformité entre cette identité et les informations portées sur l'objet,
- un dispositif d'impression et de délivrance d'un récépissé de dépôt portant, outre les données d'identification, le poids de l'objet, la date et l'heure du dépôt,
- un ensemble électronique de gestion des opérations précédentes et de transmission des opérations mémorisées à un poste centralisateur.

Application à la collecte notamment de déchets hospitaliers en conteneurs.

## Description

Réceptacle à accès personnalisé pour la collecte contrôlée d'objets d'origine spécifique, c'est-à-dire avec identification et gestion desdits objets.

A titre d'exemple non limitatif, de tels objets peuvent être des déchets médicaux contaminés du type hospitalier, qui sont déposés par des personnes exerçant des professions médicales telles que médecins, vétérinaires, chirurgiens, dentistes et analogues.

La description qui suit se réfèrera donc à l'application du dispositif selon l'invention à cette collecte, notamment dans le cadre du procédé de collecte des déchets hospitaliers faisant l'objet de la demande de Brevet Européen n°91440066.8 , déposée le 13/08/1991.

Dans ce cas, le réceptacle selon l'invention est destiné à la collecte de "conteneurs" de déchets hospitaliers, tels que décrits dans la demande ci-dessus identifiée, en vue d'assurer l'ensemble des fonctions essentielles suivantes :
1 - Accès au dépôt du conteneur sur autorisation délivrée après contrôle de l'identité du déposant et de la conformité entre cette identité et les informations portées sur le contenu.
2 - Délivrance au déposant d'un récépissé de dépôt portant, outre les données d'indentification, le poids du contenu et la date et l'heure de dépôt.
3 - Gestion de l'ensemble des opérations précédentes, avec transmission périodique des opérations mémorisées à un poste centralisateur.

Bien entendu, chacune des fonctions essentielles 1 à 3 ci-dessus comporte plusieurs composantes de détail, comme il va être indiqué ci-dessous.

En premier lieu, sur le plan mécanique, le réceptacle selon l'invention comporte une trappe d'accès verrouillable à deux positions d'ouverture, correspondant à deux phases dans l'opération de dépôt.

Dans une première phase, l'utilisateur utilise une carte à puce, un code ou tout autre moyen analogue pour obtenir l'autorisation d'accès au réceptacle. Dans l'affirmative, la validation de la carte à puce déclenche le déverrouillage de la trappe, ce qui permet le dépôt provisoire du conteneur.

La carte à puce peut remplir d'autres fonctions comme, entre autres, la fonction de carte de paiement en étant créditée ou débitée d'une certaine valeur lors de cette première phase à chaque opération.

Puis, l'ouverture de la trappe étant détectée, le dépôt du conteneur et sa présence sont également détectés, ce qui détermine la lecture des informations qu'il porte et sa pesée.

Après réalisation des différents tests, si le conteneur est accepté, l'utilisateur, dans une seconde phase, peut pousser la trappe à fond, ce qui provoque la chute du conteneur dans un casier où sont déclanchées les opérations d'édition et de délivrance à l'utilisateur d'un récépissé portant l'identiication de l'utilisateur, l'identification du conteneur, le poids du conteneur et la date et l'heure de dépôt. Au surplus, en ce point est commandé le vaporisage d'un désinfectant dans le réceptacle.

En cas de refus du conteneur, l'utilisateur récupère son conteneur et repousse la trappe pour la verrouiller, ce qui provoque la restitution de la carte.

L'ensemble des opérations est mémorisé par un circuit de gestion et de contrôle et transmis en temps réel ou périodiquement par tout moyen classique à un poste central. Il en est de même des anomalies de fonctionnement éventuelles et des alarmes provoquées par exemple, par effraction de la trappe.

L'ensemble des fonctions énumérées ci-dessus et des éléments électroniques qui les remplissent sont réunis visuellement au schéma annexé.

Bien entendu, comme déjà indiqué ci-dessus et comme il ressort de ce schéma, l'invention n'est pas limitée à la collecte des conteneurs de déchets hospitaliers. Le même matériel peut être utilisé, moyennant éventuellement les adaptations spécifiques nécessaires, pour la collecte de tous objets d'origine et/ou de destination spécifique, par exemple la collecte automatique de colis postaux.

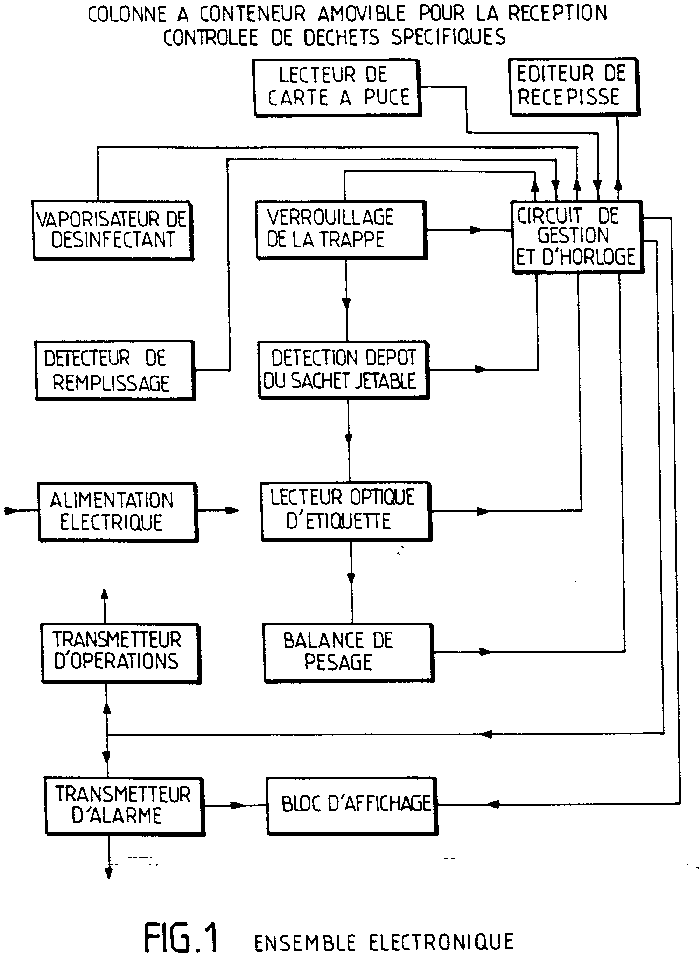

## Revendications

1. Réceptacle à accès personnalisé pour la collecte contrôlée d'objets d'origine et de destination spécifiques, caractérisé en ce qu'il comprend :
- un dispositif d'accès desdits objets sur autorisation après contrôle de l'identité du déposant et de la conformité entre cette identité et les informations portées sur l'objet,
- un dispositif d'impression et de délivrance d'un récépissé de dépôt portant, outre les données d'identification, le poids de l'objet, et la date et l'heure de dépôt.
- un ensemble électronique de gestion des opérations précédentes, et de transmission des opérations mémorisées à un poste centralisateur.

2. Réceptacle selon la revendication 1, caractérisé en ce que ledit dispositif d'accès consiste en une trappe verrouillable à deux positions d'ouverture, la première position étant accessible au moyen d'une carte à puce ou analogue, et permettant le dépôt provisoire et la pesée de l'objet, la seconde position étant accessible moyennant la conformité des informations portées par l'objet et permettant la chute de l'objet dans le réceptacle avec déclenchement des opérations d'édition et de délivrance dudit récépissé.

3. Réceptacle selon la revendication 2, caractérisé en ce qu'à la seconde position d'ouverture de la trappe, est déclenché un vaporisage de désinfectant.

4. Réceptacle selon les revendications 1 à 3, caractérisé en ce qu'il est destiné à la collecte des déchets hospitaliers.
